# EUROPÄISCHE PATENTSCHRIFT

(11) **EP 3 100 704 B1**
(45) Veröffentlichungstag und Bekanntmachung des Hinweises auf die Patenterteilung: **29.11.2017**
(21) Anmeldenummer: 16175672.1
(22) Anmeldetag: 25.07.2008
(51) Int. Cl.: A61F 2/80, A61F 2/78, A61F 2/30

(54) **PROTHESENSCHAFT UND SYSTEM AUS PROTHESENSCHAFT UND PROTHESENEINRICHTUNG**
PROSTHESIS SHAFT AND SYSTEM COMPRISING A PROSTHESIS SHAFT AND A PROSTHETIC DEVICE
TIGE DE PROTHESE ET SYSTEME DE TIGE DE PROTHESE ET DISPOSITIF DE PROTHESE

(30) Priorität: 26.07.2007 DE 102007035410
(43) Veröffentlichungstag der Anmeldung: 07.12.2016
(62) Teilanmeldung aus: 08801081.4
(73) Patentinhaber: Otto Bock HealthCare GmbH, 37115 Duderstadt (DE)
(72) Erfinder: Zarling, Sven, 37115 Duderstadt (DE); Kettwig, Thomas, 23923 Lüdersdorf OT Herrnburg (DE); Garus, Bernard, 37574 Einbeck (DE); Ruess, Felix, 2201 Gerasdorf (DE)
(74) Vertreter: Stornebel, Kai

(56) Entgegenhaltungen:
- DE-A1- 3 229 812
- FR-A- 2 512 666
- GB-A- 675 811
- GB-A- 702 696
- US-A- 4 872 879
- US-A- 5 529 575
- US-A- 5 571 209
- US-A- 5 653 766
- US-A- 5 728 165
- US-A1- 2004 158 332

## Beschreibung

Die Erfindung betrifft einen Prothesenschaft zur Aufnahme eines Amputationsstumpfes einer Extremität mit Anschlussmitteln für eine distale Protheseneinrichtung sowie ein System aus einem Prothesenschaft und einer Protheseneinrichtung.
Aus dem Stand der Technik sind verschiedene Verfahren und Konzepte bekannt, um Patienten bei Amputationen an den z.B. unteren Extremitäten mit einer Prothese zu versorgen. Bei ober- oder unterschenkelamputierten Patienten oder bei Patienten mit einer Knieexartikulation ist ein klassisches Verfahren zum Herstellen eines Prothesenschaftes das Gipsabformverfahren, bei dem von einem Patienten ein Gipsabdruck des Amputationsstumpfes genommen wird, dieser als Vorlage für ein Stumpfmodell genommen und auf dieses Stumpfmodell ein Prothesenschaft, beispielsweise ein Oberschenkelschaft geformt wird. Dieser Oberschenkelschaft besteht aus einem Kunststoff und umschließt den Amputationsstumpf vollständig. Zur Befestigung des Oberschenkelschaftes an dem Stumpf werden verschiedene Konzepte vorgeschlagen, beispielsweise wird über den Amputationsstumpf ein Liner gezogen, der an der Außenseite dichtend an dem Schaft anliegt. Über ein Ausstoßventil oder eine Pumpe wird die Luft aus dem Zwischenraum zwischen dem Liner und dem Außenschaft entfernt, so dass der Prothesenschaft mittels Unterdruck an dem Amputationsstumpf des Patienten gehalten wird. Bei einem Ausstoßventil wird bei jedem Schritt die eingetretene Luft ausgestoßen, eine aktive Evakuierung findet nicht statt. Bei einer Pinlösung befindet sich am Ende des Liners ein Dorn, der im Schaft verriegelt wird. An dem Prothesenschaft selbst sind dann weitere Protheseneinrichtungen befestigt, beispielsweise Prothesenkniegelenke und die weiteren Einrichtungen wie Verbindungselement und Prothesenfuß.

Das individuelle Anpassen eines Prothesenschaftes an den Amputationsstumpf ist außerordentlich aufwendig und zeitintensiv und bedarf eines ausgebildeten Orthopädietechnikers. Zur individuellen Anpassung des Prothesenschaftes sind mehrere Anproben notwendig, so dass zwischen der Operation und dem Fertigstellen eines solcherart angepassten Prothesenschaftes neun bis zwölf Monate vergehen können. Mit diesen Prothesenschäften kann ein hohes Maß an Stabilität und Aktivität für den Prothesennutzer bereitgestellt werden. Die Versorgung eines Patienten mit einer Amputation an einer oberen Extremität erfolgt vergleichbar.

Die mit Protheseneinrichtungen zu versorgenden Patienten haben jedoch nicht alle das Bedürfnis nach großer Mobilität. Beispielsweise an Diabetes erkrankte, ältere Personen, denen aufgrund von Diabetes induzierten Gangränen Teile der unteren Extremitäten amputiert werden mussten, sind vielfach nicht in der Lage, weite Strecken zu gehen. Darüber hinaus weist der Amputationsstumpf hohe Volumenschwankungen auf, so dass eine korrekte Anpassung des Prothesenschaftes an den Stumpf nur schwer möglich ist. Darüber hinaus mangelt es häufig an der Fähigkeit zu einer aktiven Kooperation des Patienten bei dem Anpassen des Prothesenschaftes. Gerade für solche Patienten ist es jedoch besonders wichtig, möglichst schnell mit einer Prothese versorgt zu werden, damit die Phasen der Bettlägerigkeit minimiert werden können. Bei Versorgungen der oberen Extremitäten ist es ebenfalls wichtig, möglichst schnell eine Prothese anzupassen, damit motorische Fähigkeiten nicht verloren gehen.

Die EP 1 656 911 A1 beschreibt einen Prothesenschaft mit einer geschlossenen Schale und einem lateral darin angeordneten Element, das über einen Spanngurt, der über zwei Schlitze in die Schale eingeführt wird, in Richtung auf den Stumpf gezogen wird. Der Boden der Schale ist gepolstert und weist Anschlussmittel für einen Unterschenkel auf.

Die DE 32 29 812 A1 betrifft eine Stumpfaufnahmeschale für ein künstliches Glied mit zumindest einer Schale, die einen gekrümmten, offenen Querschnitt aufweist und deren Schalenenden im angelegten Zustand einander zumindest teilweise überlappen. An der Schale sind Spannmittel angeordnet, die in Umfangsrichtung wirksam sind und die Schalenenden zueinander verspannen.

Aufgabe der vorliegenden Erfindung ist es, insbesondere für geriatrische Patienten einen Prothesenschaft und ein System aus einem Prothesenschaft und daran befestigten Protheseneinrichtungen bereitzustellen, mit denen eine schnelle Anpassbarkeit an den Patienten und eine preiswerte Versorgung möglich ist, so dass dieser möglichst schnell mobil wird oder aktiv bleibt.

Erfindungsgemäß wird diese Aufgabe durch einen Prothesenschaft mit den Merkmalen des Anspruchs 1 und ein entsprechendes System mit den Merkmalen des Anspruchs 15 gelöst. Vorteilhafte Ausgestaltungen und Weiterbildungen der Erfindung sind in den Unteransprüchen beschrieben.

Der erfindungsgemäße Prothesenschaft zur Aufnahme eines Amputationsstumpfes einer Extremität mit Anschlussmitteln für eine distale Protheseneinrichtung sieht vor, dass der Prothesenschaft zumindest eine Schale aufweist, die einen gekrümmten, offenen Querschnitt aufweist und deren Schalenenden im angelegten Zustand einander zumindest teilweise überlappen, wobei zumindest ein Spannmittel an der Schale angeordnet ist, das in Umfangsrichtung wirksam ist. Über das Spannmittel ist eine schnelle und einfache Anpassbarkeit des Prothesen- insbesondere Oberschenkelschaftes an die Kontur des Amputationsstumpfes gegeben, da durch das Spannen eine radiale Verlagerung der Schalenenden aufeinander zu bewirkt werden kann, so dass keine individuelle Abformung mehr erfolgen muss. Ebenfalls kann durch das Vorsehen des Spannmittels der Prothesenschaft und damit die gesamte Prothese schnell und einfach angelegt und auch wieder abgelegt werden, wodurch die Akzeptanz zum Tragen der Prothese erhöht wird. Dadurch ist es möglich, auch frisch operierte Patienten schnell mit einer Prothese zu versorgen, so dass der Zeitraum der eingeschränkten Mobilität oder Aktivität verringert wird. Die Schale kann beispielsweise spiralartig ausgebildet sein, wobei sich über das oder die Spannmittel der Umfang des Schaftes verändern lässt. Ebenfalls können zwei einander gegenüberliegende Schalenbereiche einander überlappen, so dass statt einer drehenden Überlappbewegung eine klappende Überlappbewegung bei einer Veränderung der effektiven Länge des Spannmittels oder der Spannmittel stattfindet. Die Schale kann einstückig ausgebildet sein. Die Schale oder Teilschalen sind bevorzugt aus einem formstabilen Kunststoff gefertigt, um einen möglichst leichten Prothesenschaft bereitstellen zu können. Die Schale oder Teilschalen weisen dabei Bereiche unterschiedlicher Elastizität auf, um einerseits den Stabilitätsanforderungen zu genügen und andererseits ein bequemes Tragen zu ermöglichen. So ist beispielsweise im proximalen Bereich der Schale oder Teilschalen diese flexibel ausgebildet, insbesondere in Frontal- und Dorsalrichtung, so dass ein Sitzen bei angelegter Prothese nicht als unangenehm empfunden wird. Die Bereiche unterschiedlicher Flexibilität können durch die Verwendung von Werkstoffen mit unterschiedlichen physikalischen Eigenschaften oder durch unterschiedliche Materialstärken ausgebildet werden. Die flexibleren Bereiche sind dabei in der Regel dünner als die steiferen Bereiche ausgebildet oder können aus einem flexibleren Material als der Rest der Schale ausgebildet sein, beispielsweise durch ein 2-Komponenten Spritz- oder Gießverfahren.

Um einerseits das Hineingleiten des mit einem Liner versehenen Amputationsstumpfes zu erleichtern und andererseits eine stabile Zuordnung von Amputationsstumpf und Prothesenschaft zu gewährleisten, ist vorgesehen, dass die Innenseite der Schale oder Teilschalen mit einer richtungsabhängigen Oberflächengestaltung, z.B. einem Strichvelours ausgestattet oder beschichtet ist, der eine Orientierung aufweist, die ein leichtes Einführen ermöglicht, jedoch einen erhöhten Widerstand entgegen der Einführrichtung bereitstellt. Die Oberflächengestaltung, z.B. Fasern sind dazu in distaler Richtung geneigt, während die Außenseite des Liners eine Ausgestaltung oder Beschichtung in einem entgegengesetzt orientierten Strich aufweist oder mit einer mit der Oberflächengestaltung oder dem Velours entsprechend zusammenwirkenden Oberfläche ausgestattet ist. Die Ausgestaltung des Schaftes und des Liners mit richtungsabhängiger Oberflächengestaltung ist auch unabhängig von der Konstruktion des Schaftes bei anderen Ausgestaltungen des Schafte einsetzbar und stellt eine unabhängige Lösung des Problems bereit, einen Liner in einem Schaft zu halten.

Die Schale kann aus mehreren Teilschalen aufgebaut sein, die zueinander verlagerbar ausgebildet sind. Zwei oder mehrere Teilschalen können zu einer Gesamtschale kombiniert werden, indem die Teilschalen blattartig einander zumindest teilweise überlappend angeordnet sind. Ebenfalls können die Teilschalen an ihren distalen Abschnitten miteinander verbunden oder aneinander angeformt sein, so dass sich eine elastische Lagerung mehrerer schalenartiger Abschnitte an einem gemeinsamen Bereich ausbildet.

Bevorzugt ist die Schale oder sind die Teilschalen medial und lateral an dem Amputationsstumpf angeordnet und weisen je zumindest ein Anschlussmittel für die sich distal anschließende Protheseneinrichtung auf. Durch die Medial/Lateralanordnung ist es möglich, die Überlappung der Schalenenden oder Teilschalen frontal und dorsal anzuordnen, um dort eine erhöhte Stabilität zu erhalten. Darüber hinaus ist es mit dieser Anordnung möglich, die Anschlussmittel zur Befestigung der Protheseneinrichtung medial und lateral vorzusehen. Eine alternative Ausgestaltung sieht vor, dass eine unilaterale Anbindung erfolgt, die entweder eine mediale oder laterale Anordnung der Anschlussmittel zulässt, wodurch sich der Gestaltungsspielraum für die Prothese insgesamt erhöhen lässt. Dadurch können individuelle Vorlieben oder Notwendigkeiten leichter berücksichtigt werden.

Dabei ist die Schale oder sind die Teilschalen medial und lateral steif ausgebildet und weisen einen hohen Verformungswiderstand insbesondere gegen eine Biegung um eine Achse in Frontal- oder Gehrichtung auf, um eine ausreichende Stabilität bereitstellen zu können. Die Steifigkeit wird durch eine Krümmung der Schale oder Teilschalen unterstützt. Eine Anbindung der Schale oder der Schalen an den Rahmen kann auch einseitig erfolgen. Bei einer unilateralen Anbindung kann diese medial oder lateral ausgeführt sein.

Eine erleichterte Anpassung und Überlappung der Schale oder Teilschalen ist gegeben, wenn diese mit einer Krümmung versehen ist bzw. sind, so dass diese ineinander geschoben oder gesteckt werden können. Dabei ist es vorgesehen, dass eine Teilschale einen größeren Krümmungsradius als die andere Teilschale aufweist, so dass eine Teilschale von der anderen an beiden Enden außen überlappt werden kann. Eine ungefähre Vorformung an die Stumpfform kann ebenfalls erfolgen.

Die Anschlussmittel zur Befestigung der distalen Protheseneinrichtung sind bevorzugt im proximalen, steifen Bereich der Schale oder Teilschalen angeordnet, insbesondere bei Oberschenkelamputationen sind die Anschlussmittel im Bereich des Trochanter major positioniert. Eine Weiterbildung der Erfindung sieht vor, dass das Drehzentrum der Anschlussmittel im Bereich der Trochanter major angeordnet ist, während die Anschlussmittel selbst an einem anderen Teil der Schale oder der Teilschalen befestigt sind. Beispielsweise kann über entsprechend geschwungene Führungen der Drehpunkt auf den Trochanter major verschoben werden.

Die Anschlussmittel können zur schwenkbaren Lagerung der Protheseneinrichtung angeordnet bzw. ausgebildet sein, wobei die Schwenkbarkeit nur für einen geringen Winkelbereich vorgesehen ist, um den Prothesenaufbau, also die Ausrichtung der Prothesenelemente zueinander und zu dem Körper, zu erleichtern. Nach dem einmal eingestellten Prothesenaufbau kann die Protheseneinrichtung über Fixiereinrichtungen, beispielsweise Schrauben oder Steckstifte, in der korrekten Position fixiert werden. Die Fixiereinrichtung kann dabei in einem Langloch geführt sein, beispielsweise in einem gekrümmten Langloch, um den Prothesenaufbau zu erleichtern.

Grundsätzlich ist es vorgesehen, dass die Schale oder die Teilschalen im angelegten Zustand bzw. im miteinander verbundenen Zustand sowohl proximal als auch distal eine Öffnung ausbildet bzw. ausbilden. Um diese Öffnung zu schließen, ist als distaler Abschluss an der Schale oder zumindest einer Teilschale eine Kappe befestigt, insbesondere lösbar befestigt, um das distale Ende des Amputationsstumpfes zu schützen.

Alternativ oder ergänzend ist es vorgesehen, dass die Innenseite der Teilschalen mit einer Haftschicht, beispielsweise aus Silikon, Polyurethan oder einem Copolymer beschichtet sind, gegebenenfalls nur bereichsweise mit einer Haftbeschichtung versehen sind, um an einem mit einer entsprechenden Außenschicht versehenen Liner besser haften zu können.

Als Spannmittel können Klettbänder, Skistiefelverschlüsse, Laschen oder Riemen vorgesehen sein, ebenfalls können alternative Befestigungsmittel zum variablen Einstellen des Umfanges der Schale oder der Abstände oder Überlappungen der Teilschalen zueinander sowie zur Befestigung der Teilschalen oder der Schalenenden aneinander vorgesehen sein. Die Spannmittel sind bevorzugt auf der Außenseite des Schaftes angeordnet und geführt, um einen unmittelbaren Kontakt mit dem Stumpf und damit eine Gefahr des Einklemmens des Stumpfes zu vermeiden.

Die Spannmittel können frontal geführt sein, insbesondere sind die Verschlusseinrichtungen oder Hebel frontal angeordnet, um ein Anlegen bzw. Ablegen der Prothese und des Prothesenschaftes zu erleichtern. Bevorzugt wirkt das Spannmittel oder wirken die Spannmittel zirkulär.

Zur individuellen Anpassung der Schale oder Teilschalen an die Stumpfkontur sind zumindest zwei Spannmittel axial zueinander versetzt an dem Prothesenschaft angeordnet, wodurch sich ein sich proximal erweiternder Kegel ausbilden lässt. Ebenfalls können die Volumenschwankungen, die über die Länge des Amputationsstumpfes unterschiedlich ausgeprägt sein können, über mehrere Spannmittel besser ausgeglichen werden. Auch kann das Spannmittel mit zwei axial versetzt angreifenden Führungen angeordnet werden.

Die an dem Prothesenschaft befestigbare Protheseneinrichtung weist einen Rahmen auf, der bevorzugt lateral und/oder medial an dem Prothesenschaft befestigt ist, wobei der Rahmen bevorzugt als Außenrahmen ausgebildet ist, der beispielsweise aus einem Metallprofil gefertigt ist. Eine einseitige Befestigung kann ausreichend sein, wobei bevorzugt eine mediale Befestigung gewählt wird. An dem Rahmen können dann die weiteren Protheseneinrichtungen befestigt werden, beispielsweise ein Prothesenkniegelenk oder dergleichen. Der Prothesenschaft und die Protheseneinrichtung, die je nach Amputationshöhe unterschiedlich ausgebildet sein kann, bilden ein System zur prothetischen Versorgung eines Patienten.

Nachfolgend werden Ausführungsbeispiele der Erfindung anhand der beigefügten Figuren näher erläutert. Es zeigen:
- Figur 1 -: ein System aus Prothesenschaft und Protheseneinrichtung im angelegten Zustand;
- Figur 2 -: eine isolierte Darstellung des Prothesenschaftes und einer daran befestigten Protheseneinrichtung;
- Figur 3 -: eine Detaildarstellung eines zusammengefügten Prothesenschaftes;
- Figur 4 -: einen Prothesenschaft mit separaten Teilschalen;
- Figur 5 -: einen Prothesenschaft in Explosionsdarstellung;
- Figur 6 -: eine Detaildarstellung einer Teilschale mit einem montierten Rahmen;
- Figur 7 -: eine Seitenansicht einer montierten Teilschale;
- Figur 8 -: eine verschwenkte Teilschale;
- Figur 9 -: eine Variante der Schaftausbildung in offener Stellung; sowie
- Figur 10 -: ein Prothesenschaft nach Figur 9 in geschlossener Stellung.

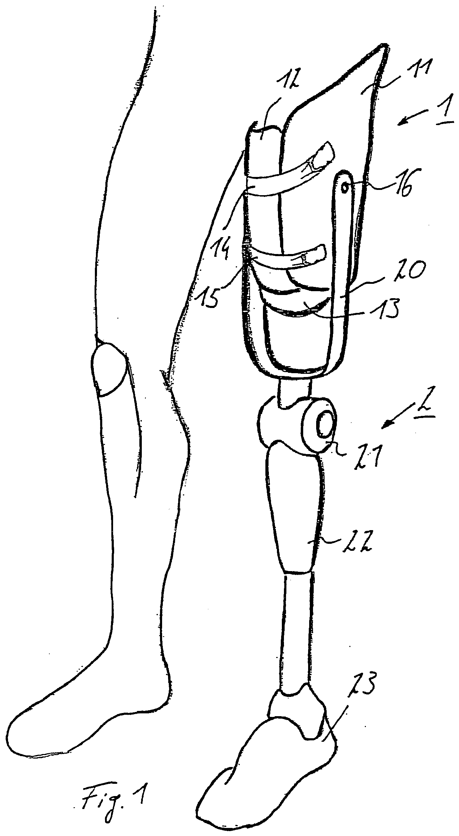

In der Figur 1 ist ein System aus einem Prothesenschaft 1, vorliegend ein Oberschenkelschaft, sowie einer daran befestigten Protheseneinrichtung 2 in einem angelegten Zustand gezeigt, das gesunde rechte Bein ist schematisch angedeutet. Der Prothesenschaft 1 besteht in dem Ausführungsbeispiel aus zwei Teilschalen 11, 12, die einander überlappend um den Amputationsstumpf herum gelegt sind. Über zwei axial zueinander versetzt an den Teilschalen 11, 12, befestigte Spannmittel 14, 15 ist der Prothesenschaft 1 an den Umfang und die Kontur des Amputationsstumpfes angepasst. Zwischen dem Prothesenschaft 1 und dem Amputationsstumpf ist ein nicht dargestellter Liner angeordnet, der über den Amputationsstumpf gezogen ist und einen unmittelbaren Kontakt der Teilschalen 11, 12 über die gesamte Fläche mit der Haut des Amputationsstumpfes vermeidet. Durch den Liner wird eine mechanische Kopplung zwischen dem Prothesenschaft 1 und dem Amputationsstumpf hergestellt.

Die beiden Teilschalen 11, 12 sind aus einem flächigen, formstabilen Kunststoff gefertigt und weisen eine Krümmung auf, so dass sie möglichst großflächig an dem Amputationsstumpf bzw. dem Liner anliegen. In der dargestellten Ausführungsform wird die mediale Teilschale 12 von der lateralen Teilschale 11 außen im frontalen Bereich überdeckt. Eine entsprechende Überdeckung kann auch im dorsalen Bereich stattfinden, was bedeutet, dass der Krümmungsradius der lateralen Teilschale 11 größer als der der medialen Teilschale 12 ist. Die separaten Teilschalen 11, 12 weisen einen offenen Querschnitt auf und bilden im angelegten Zustand eine mehrteilige Hülse, die proximal und distal offen ist. Das distale Ende des Prothesenschaftes 1 ist vorliegend durch eine Kappe 13 verschlossen, die an den Teilschalen 11, 12 befestigt ist. Die Kappe 13 dient zum Schutz des distalen Endes des Amputationsstumpfes. Grundsätzlich ist auch die Möglichkeit gegeben, dass diese Kappe 13 an dem Liner befestigt oder ausgebildet ist.

An den Teilschalen 11, 12 sind im proximalen Bereich des Prothesenschaftes 1 Anschlussmittel 16 zur Befestigung eines Rahmens 20 vorgesehen. Die Anschlussmittel 16, die als Schraubverbindung ausgestaltet sind, sind nur auf der lateralen Seite dargestellt, tatsächlich jedoch an beiden Teilschalen 11,12 vorhanden. Der Rahmen 20 ist als U-förmiger Bügel aus einem Metall- oder Kunststoffprofil ausgebildet und dient als Außenrahmen, um den Prothesenträger möglichst wenig zu belasten. Die beiden Bügel des U-förmigen Außenrahmens 20 sind medial und lateral angeordnet.

An dem Rahmen 20 sind sich distal anschließend weitere Komponenten der Protheseneinrichtung 20 angeordnet, vorliegend ein Prothesenkniegelenk 21, ein Verbindungselement 22 sowie ein Prothesenfuß 23. Die Protheseneinrichtung 2 insgesamt ist bevorzugt als ein Sperrkniegelenk oder Kniegelenk mit hoher Sicherheit ausgebildet, das eine einfache Konstruktion aufweist und vor allen Dingen sicherstellt, dass kein ungewolltes Einknicken des Prothesenkniegelenkes 21 auftritt. Sofern das System nicht zur Versorgung geriatrischer Patienten eingesetzt wird, sondern als eine Erstversorgung, können auch andere Konstruktionen des Prothesenkniegelenkes 21 vorgesehen sein.

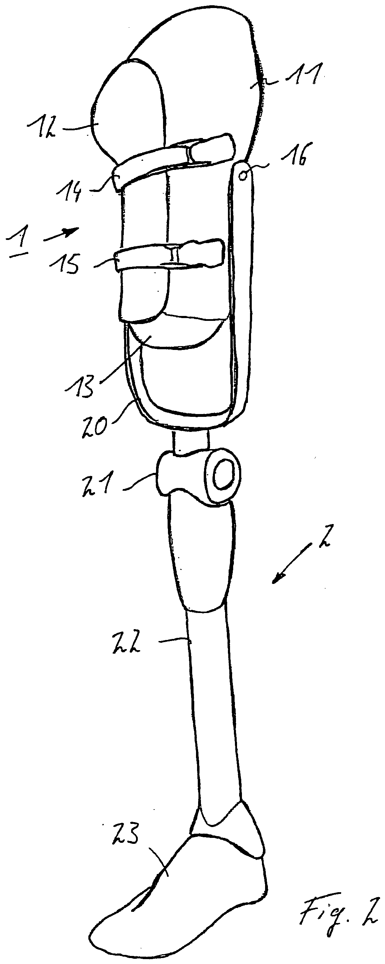

In der Figur 2 ist das System aus Prothesenschaft 1 und Protheseneinrichtung 2 separat dargestellt. Im Gegensatz zu der Figur 1 ist die mediale Teilschale 12 hier außenliegend angeordnet und überlappt die laterale Teilschale 11. Die Spannmittel 14, 15, die als Riemen, Klettband oder beispielsweise Skistiefelverschluss ausgebildet sein können, können entweder nur an einer Teilschale 11, 12 befestigt sein und in Umfangsrichtung wirken, so dass die Teilschalen 11, 12 aufeinander zu bewegt werden, oder die Verbindung zwischen den beiden Teilschalen 11, 12 herstellen, so dass ein Endpunkt eines Spannmittels 14, 15 an der medialen Teilschale 12 und ein zweiter Endpunkt an der lateralen Teilschale 11 angeordnet ist.

Da das System aus Prothesenschaft 1 und Protheseneinrichtung 2, insbesondere für weniger aktive Patienten geeignet erscheint, die häufig sitzen, ist es zur Vermeidung von Druckstellen und zur Erhöhung des Tragekomforts vorgesehen, dass der Prothesenschaft 1 in den frontalen und dorsalen Bereichen, insbesondere im proximalen Bereich der Teilschalen 11, 12 flexibel und weich ausgebildet ist, während der distale Bereich der Teilschalen 11, 12, insbesondere medial und lateral stabil ist. Insbesondere der Bereich der Anschlussmittel 16 ist stabil, um die beim Gehen oder Stehen auftretenden Kräfte in den Prothesenschaft 1 einleiten zu können. Die Anschlussmittel 16 sind möglichst proximal an den Teilschalen 11, 12 befestigt, bevorzugt im Bereich des Trochanter major bei Ausbildung des Prothesenschaftes 1 als Oberschenkelschaft.

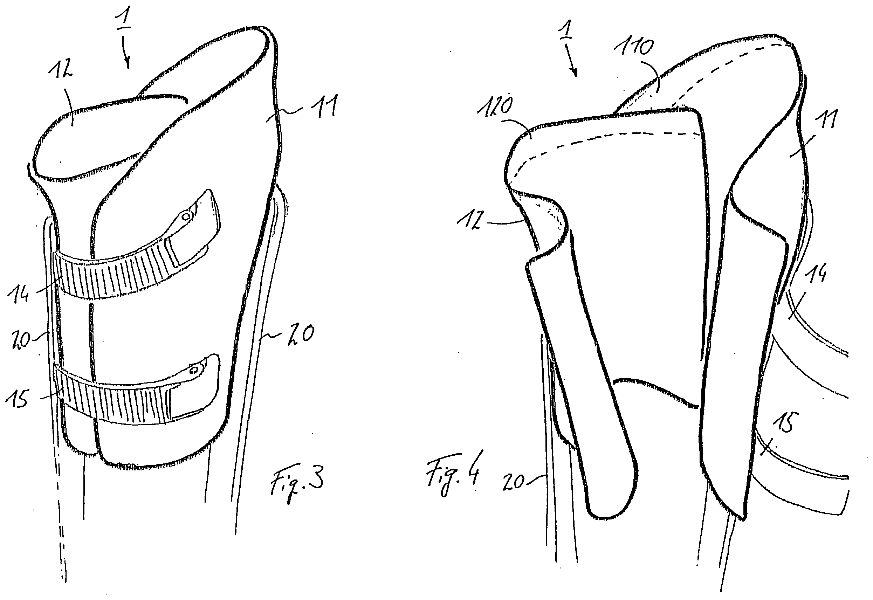

In der Figur 3 ist in vergrößerter Einzeldarstellung der Prothesenschaft 1 mit den beiden Teilschalen 11, 12 und den Spannmitteln 14, 15 dargestellt, die über Formschlusseinrichtungen den Umfang des Prothesenschaftes 1 einstellbar ausgestalten. Die vorgeformten Teilschalen 11, 12 weisen ein ausreichendes Maß an Flexibilität bei gleichzeitiger Stabilität auf, um durch Verändern der Spannung in den Spannmitteln 14, 15 eine hinreichend genaue Anpassung an den Amputationsstumpf bereitzustellen. Der Außenrahmen 20 mit den Bügeln ist medial und lateral befestigt, insbesondere durch eine Schraubverbindung.

In der Figur 4 ist der Prothesenschaft 1 aufgeklappt dargestellt. Es ist zu erkennen, dass die mediale Teilschale 12 in der lateralen Teilschale 11 aufgenommen ist und von dieser frontal und dorsal überdeckt wird. In den proximalen Bereichen 110, 120 der Teilschalen 11, 12 sind Zonen geringerer Festigkeit und erhöhter Flexibilität ausgebildet, die das Sitzen erleichtern. Ebenfalls kann die Wandstärke in diesen Bereichen 110, 120 verringert sein, um ein Sitzen bei angelegter Prothese zu erleichtern. Die Spannmittel 14, 15 sind geöffnet dargestellt. Durch das Öffnen der Spannmittel 14, 15 kann der Prothesenschaft 1 geöffnet werden, so dass der Prothesenträger sehr leicht den Prothesenschaft 1 anlegen kann, indem er in den geöffneten Prothesenschaft 1 hineinsteigt, die Teilschalen 11, 12 ineinander schiebt und die Spannmittel 14, 15 nach Bedarf schließt. Durch die variable Ausgestaltung der Spannmittel 14, 15 ist es möglich, Volumenänderungen des Amputationsstumpfes aufzufangen und stets eine ausreichend sichere Festlegung des Prothesenschaftes 1 an dem Amputationsstumpf, insbesondere an dem Liner zu ermöglichen.

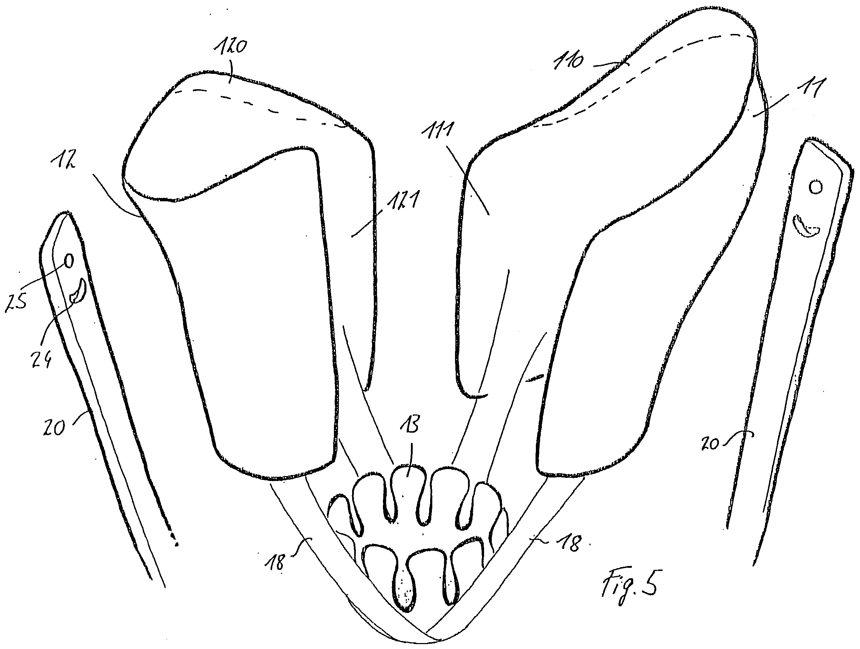

In der Figur 5 ist der Prothesenschaft 1 in Explosionsdarstellung gezeigt. Die laterale Teilschale 11 ist so ausgeformt, dass sie den Trochanter major überdeckt, während die mediale Teilschale 12 so ausgebildet ist, dass das Sitzbein nicht überdeckt wird oder nur mit dem flexiblen Bereich 120 in Kontakt tritt. An den distalen Enden der Teilschalen 11, 12 ist die Kappe 13 über Befestigungsbänder 18, beispielsweise Klettbändern, befestigt. Die Befestigungsbänder 18 sind kreuzweise angeordnet und halten die Kappe 13, die an ihrem offenen Rand elastische Finger ausbildet, an dem Prothesenschaft 1.

Die Innenseite 111, 121 der Teilschalen 11, 12 können mit unterschiedlichen Beschichtungen versehen sein, um ein Anhaften an dem Liner bzw. eine Kopplung zwischen Liner und Teilschalen 11, 12 zu bewirken. Die Beschichtung 111, 121 kann beispielsweise als ein Strichvelours oder ein anderes Gewebe mit einer entsprechenden Ausrichtung der Fasern ausgebildet sein, die ein Einführen des Liners und des Amputationsstumpfes in den im Wesentlichen rohrförmigen Prothesenschaft 1 zu erleichtern und gleichzeitig ein Herausrutschen zu verhindern. Dazu können die Fasern oder Haken in Richtung des distalen Randes geneigt angeordnet sein, so dass eine Einführbewegung erleichtert wird, während eine entgegengesetzte Bewegung blockiert bzw. erschwert wird. Zum Lösen des Schaftes 1 von dem Liner werden dann die Spannmittel 14, 15 geöffnet und der Prothesenschaft 1 zusammen mit der Protheseneinrichtung 2 abgenommen. Ebenfalls ist es möglich, die Innenseite der Teilschalen 11, 12, mit einem Copolymer oder Silikon zu beschichten, um ein Anhaften des Liners, der auf der Außenseite mit einer entsprechenden Beschichtung versehen ist, zu unterstützen.

In der Figur 5 ist weiterhin der Außenrahmen 20 dargestellt, der Bohrungen 25 an dem proximalen Ende aufweist, durch das die Anschlussmittel 16 in Gestalt von Schrauben oder Bolzen hindurch geführt werden. Ebenfalls sind gekrümmte Langlöcher 24 in dem Rahmen 20 ausgebildet, die Mittel zum Fixieren des Prothesenschaftes 1 relativ zu dem Rahmen 20 aufnehmen.

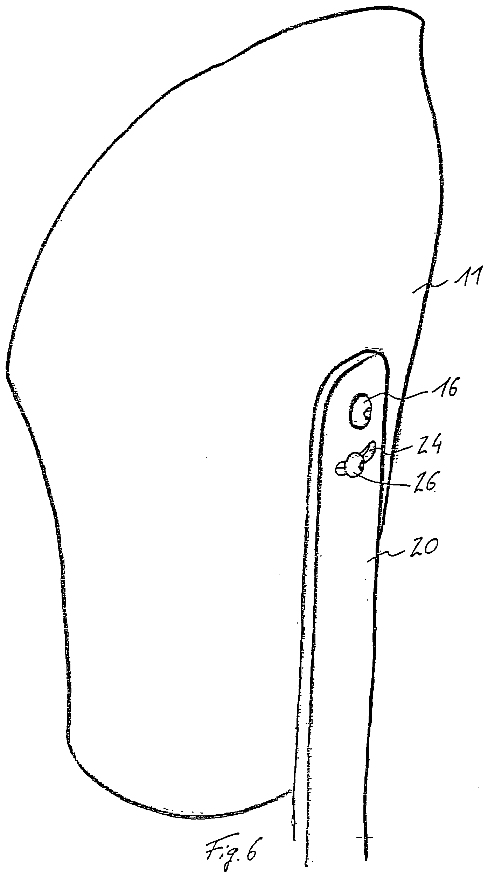

In der Figur 6 ist eine solche Ausgestaltung im montierten Zustand gezeigt. An dem Rahmen 20 ist vorliegend die laterale Teilschale 11 über einen Schraubbolzen 16 als Anschlussmittel befestigt. Weiterhin ist in dem gekrümmten Langloch 24 eine weitere Schraube 26 geführt, die durch das Langloch 24 hindurch ragt und in der lateralen Teilschale 11 eingeschraubt ist. In dem Kunststoff der Teilschale 11 können entsprechende Gewinde eingearbeitet und einlaminiert sein, um eine stabile Befestigung des Rahmens 20 an der Teilschale 11 zu bewirken.

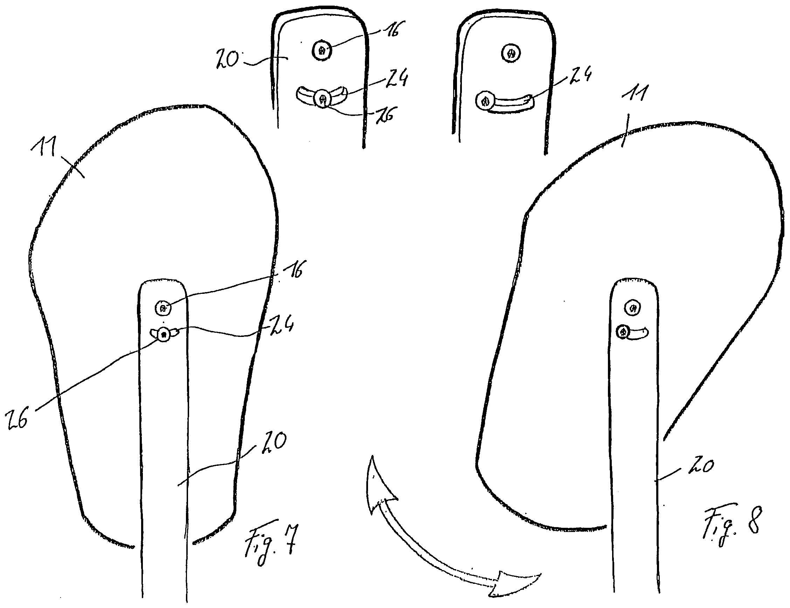

Die Figur 7 zeigt den Zustand gemäß Figur 6 in Seitenansicht. In der Figur 8 ist gezeigt, dass der Prothesenschaft 1, vorliegend die Teilschale 11, relativ zu dem Rahmen 20, um das Anschlussmittel 16 in Gestalt einer Schraube schwenkbar ist, was durch den Doppelpfeil angedeutet wird. Innerhalb des Langloches 24 gleitet die Schraube 26 hin und her, bis eine optimale Orientierung des Prothesenschaftes zu dem Rahmen 20 gefunden ist. Dann wird die Schraube 26 angezogen und der Oberschenkelschaft 1 relativ zu dem Rahmen 20 fixiert. Eine solche Einstellbarkeit ist notwendig, da die Hüftmuskulatur zu einer Verkürzung neigt, wenn keine Streckung erfolgt. Bei überwiegend sitzender Tätigkeit wird die Hüftbeugemuskulatur kontrakt, gleiches gilt, wenn aufgrund der Amputation ein Gegengewicht fehlt. Um diese Beugung ausgleichen zu können und weiterhin einen korrekten Prothesenaufbau bereitstellen zu können, ist die Verschwenkbarkeit und Einstellbarkeit vorteilhaft.

Mit dem System aus dem Prothesenschaft 1 und der Protheseneinrichtung 2 ist ein bequemes Sitzen möglich, ebenfalls kann ein leichtes Anlegen und Ablegen der Prothese erfolgen. Die Anpassung an ein schwankendes Stumpfvolumen kann leicht erfolgen, ebenfalls entfallen eine langwierige Anprobe und ein Abformen eines Stumpfes an dem Patienten. Die Gelenkeinrichtung 21 kann beispielsweise das Hinsetzen und Aufstehen unterstützen, indem verschiedene Sperrmodi und Dämpfungsstufen eingestellt werden können. Ebenfalls ist es möglich, das Prothesengelenk 21 als Sperrkniegelenk auszubilden. Neben der Versorgung geriatrischer Patienten kann das System zur schnellen Erstversorgung eingesetzt werden, um Patienten möglichst kurz einer Phase der Immobilität aussetzen zu müssen. Aufgrund der einfachen Anpassbarkeit können Anpassungen an das Stumpfvolumen, die aufgrund des Heilungsprozesses rückgängig werden können, schnell und einfach vorgenommen werden. Die Anpassung erfolgt dabei über eine Verschiebung der separaten Teilschalen 11, 12 zueinander, um so die sich verändernden Umfänge des Amputationsstumpfes auffangen zu können.

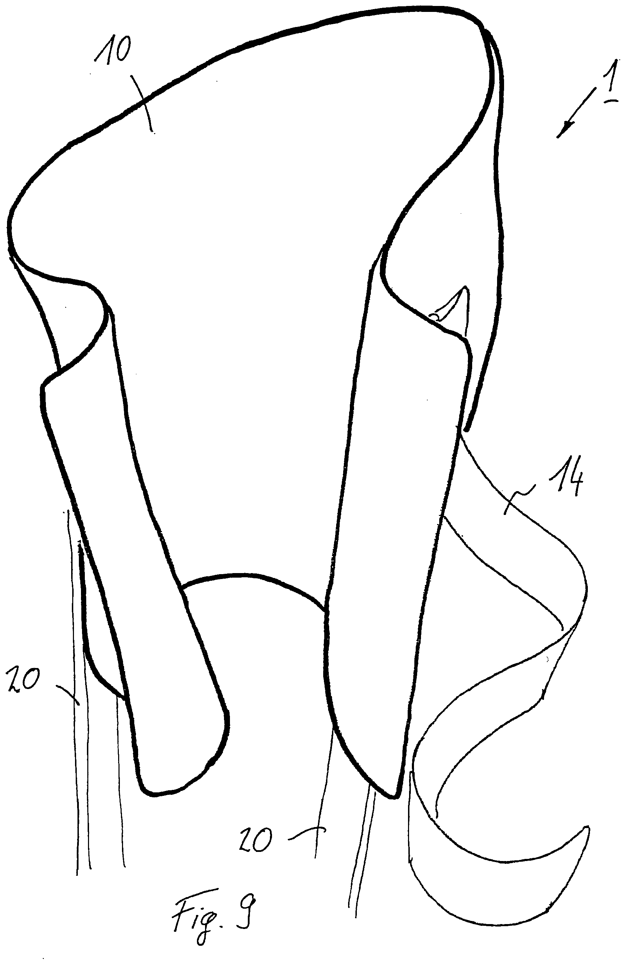

Die Figur 9 zeigt in einer perspektivischen Darstellung eine Variante der Erfindung mit einer einteiligen Schale 10, die einen offenen Querschnitt aufweist und in einer geöffneten Stellung gezeigt ist. Die Öffnung ist dabei frontal ausgerichtet, so dass der Prothesenschaft 1 leicht angelegt werden kann, indem der Prothesenschaft 10 geöffnet abgelegt wird und der mit einem Liner versehene Stumpf in den geöffneten Prothesenschaft bzw. in die geöffnete Schale 10 eingelegt werden kann. Dazu können die Schalenenden auseinander gezogen werden und sich leicht nach außen auswölben, um so ein leichtes Einführen zu ermöglichen. Die Schalenenden werden dann übereinander gelegt, vorliegend das linke Schalenende unter das rechte, laterale Schalenende, so dass frontal eine Überlappung erreicht wird. Anschließend wird das Spannmittel 14 beispielsweise durch eine Schlaufe geführt und zurückgeschlagen, um eine Befestigung zu ermöglichen. Die Befestigung kann dabei über Klettverschlüsse oder sogenannte Skischuhverschlüsse erfolgen.

Der Figur 9 ist zu entnehmen, dass das distale Ende der Schale 10 offen ist, so dass eine leichte Anpassung an unterschiedliche Stumpflängen erfolgen kann, ohne dass die Festlegung der Schale 10 an der korrekten Stelle beeinträchtigt wäre.

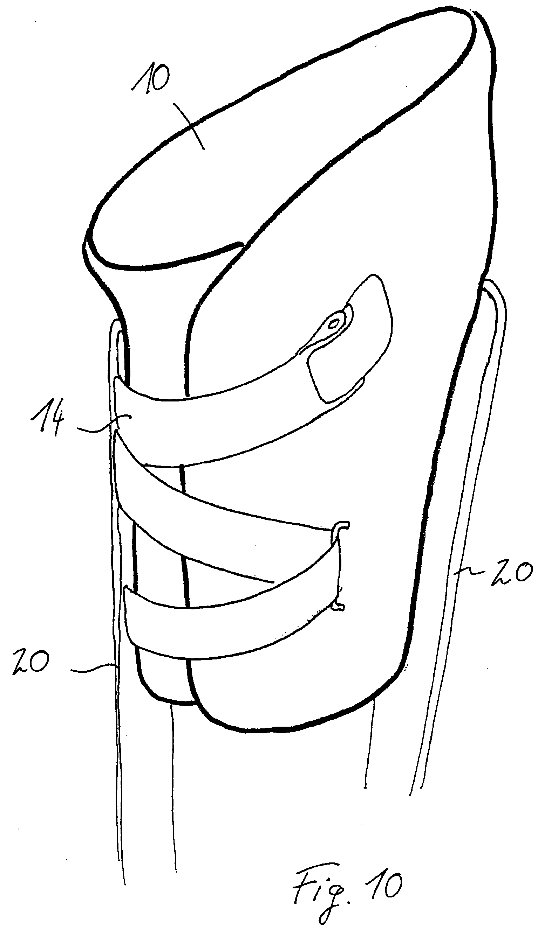

In der Figur 10 ist das Ausführungsbeispiel gemäß Figur 9 in einer geschlossenen Darstellung gezeigt, bei der das linke Schalenende unterhalb des rechten Schalenendes gelegt ist. Das Spannmittel 14 ist zickzackartig an der Schale 10 des Prothesenschaftes festgelegt, an den Wendepunkten in einer Öse geführt. Die Festlegung des Spannmittels 14 an der Außenseite der Schale 10 kann über Klettverschlüsse erfolgen, ebenfalls kann eine alternative Befestigung vorgesehen sein. Ein Spannen kann an einem endseitig angeordneten Kniehebelmechanismus oder einem Skistiefelverschluss erfolgen. Auch der Figur 10 ist zu entnehmen, dass das distale Ende des Prothesenschaftes offen ist, das Ende kann durch eine Abschlusskappe verschlossen oder begrenzt werden. Die Schale 10 weist eine spiralartige Anordnung auf, so dass bei einer Umfangsverstellung die Schalenenden spiralartig umeinander gleiten bzw. sich das rechte Ende der Schale 10 über das innen liegende, linke Schalenende schiebt.

## Patentansprüche

1. Prothesenschaft zur Aufnahme eines Amputationsstumpfes einer Extremität mit Anschlussmitteln für eine distale Protheseneinrichtung (2), wobei der Prothesenschaft (1) zumindest eine Schale (10; 11, 12) aufweist, die einen gekrümmten, offenen Querschnitt aufweist und deren Schalenenden im angelegten Zustand einander zumindest teilweise überlappen und zumindest ein Spannmittel (14, 15) an der Schale (10; 11, 12) angeordnet ist, das in Umfangsrichtung wirksam ist und die Schalenenden zueinander verspannt, wobei die Schale (10; 11, 12) aus einem formstabilen Kunststoff mit Bereichen unterschiedlicher Elastizität ausgebildet ist, **dadurch gekennzeichnet, dass** die Innenseite (111, 121) der Schale (11, 12) eine richtungsabhängige Oberflächengestaltung aufweist, die entgegen einer Einführrichtung des Amputationsstumpfes einen höheren Widerstand als in Einführrichtung bereitstellt

2. Prothesenschaft nach Anspruch 1, **dadurch gekennzeichnet, dass** die Schale (11, 12) aus mehreren Teilschalen besteht.

3. Prothesenschaft nach Anspruch 1 oder 2, **dadurch gekennzeichnet, dass** die Schale (10; 11, 12) medial und lateral zu dem Amputationsstumpf angeordnet ist und zumindest ein Anschlussmittel (16) für die distale Protheseneinrichtung (2) aufweist

4. Prothesenschaft nach einem der voranstehenden Ansprüche, **dadurch gekennzeichnet, dass** die Schale (10; 11, 12) im proximalen Bereich (110, 120) flexibel ausgebildet ist.

5. Prothesenschaft nach einem der voranstehenden Ansprüche, **dadurch gekennzeichnet, dass** die Schale (10; 11, 12) in medialer und lateraler Richtung steif ausgebildet ist.

6. Prothesenschaft nach einem der voranstehenden Ansprüche, **dadurch gekennzeichnet, dass** die Anschlussmittel (16) im proximalen, steifen Bereich der Schale (10; 11, 12) angeordnet ist.

7. Prothesenschaft nach einem der voranstehenden Ansprüche, **dadurch gekennzeichnet, dass** die Anschlussmittel (16) im Bereich des Trochanter major angeordnet sind.

8. Prothesenschaft nach einem der voranstehenden Ansprüche, **dadurch gekennzeichnet, dass** das Drehzentrum der Anschlussmittel (16) im Bereich des Trochanter major angeordnet ist.

9. Prothesenschaft nach einem der voranstehenden Ansprüche, **dadurch gekennzeichnet, dass** die Anschlussmittel (16) zur schwenkbaren Lagerung der Protheseneinrichtung (2) ausgebildet sind und dass Fixiereinrichtungen (26) zur Ausrichtung der Protheseneinrichtung (2) relativ zu dem Prothesenschaft (1) vorgesehen sind.

10. Prothesenschaft nach einem der voranstehenden Ansprüche, **dadurch gekennzeichnet, dass** an der Schale (10; 11, 12) als distaler Abschluss eine Kappe (13) lösbar befestigt ist.

11. Prothesenschaft nach Anspruch 1, **dadurch gekennzeichnet, dass** die Oberflächengestaltung als Strichvelours ausgebildet ist, der in distaler Richtung orientiert ausgerichtet ist.

12. Prothesenschaft nach einem der voranstehenden Ansprüche, **dadurch gekennzeichnet, dass** ein Spannmittel (14, 15) mit zwei axial versetzt angreifenden Führungen an der Schale (10; 11, 12) angeordnet ist.

13. Prothesenschaft nach einem der voranstehenden Ansprüche, **dadurch gekennzeichnet, dass** die Protheseneinrichtung (2) einen Rahmen (20) aufweist, der lateral und/oder medial an dem Prothesenschaft (1) befestigbar ist.

14. Prothesenschaft nach Anspruch 13, **dadurch gekennzeichnet, dass** der Rahmen (20) als Außenrahmen ausgebildet ist.

15. System aus Prothesenschaft (1) und Protheseneinrichtung (2) nach einem der voranstehenden Ansprüche.

## Claims

1. Prosthesis socket for receiving an amputation stump of an extremity, with connecting means for a distal prosthesis device (2), the prosthesis socket (1) comprising at least one shell (10; 11, 12), which has a curved, open cross section and of which the shell ends, in the applied state, at least partially overlap each other, and at least one tensioning means (14, 15) being arranged on the shell (10; 11, 12), which tensioning means (14, 15) acts in the circumferential direction and tensions the shell ends relative to each other, wherein the shell (10; 11, 12) is made from a dimensionally stable plastic with areas of different elasticity, **characterized in that** the inner face (111, 121) of the shell (11, 12) has a direction-dependent surface configuration that offers greater resistance counter to a direction of insertion of the amputation stump than it does in the direction of insertion.

2. Prosthesis socket according to Claim 1, **characterized in that** the shell (11, 12) is made of several partial shells.

3. Prosthesis socket according to Claim 1 or 2, **characterized in that** the shell (10; 11, 12) is arranged medially and laterally with respect to the amputation stump and comprises at least one connecting means (15) for the distal prosthesis device (2).

4. Prosthesis socket according to one of the preceding claims, **characterized in that** the shell (10; 11, 12) is flexible in the proximal area (110, 120).

5. Prosthesis socket according to one of the preceding claims, **characterized in that** the shell (10; 11, 12) is stiff in the medial and lateral directions.

6. Prosthesis socket according to one of the preceding claims, **characterized in that** the connecting means (16) are arranged in the proximal, stiff area of the shell (10; 11, 12).

7. Prosthesis socket according to one of the preceding claims, **characterized in that** the connecting means (16) are arranged in the area of the greater trochanter.

8. Prosthesis socket according to one of the preceding claims, **characterized in that** the center of rotation of the connecting means (16) is arranged in the area of the greater trochanter.

9. Prosthesis socket according to one of the preceding claims, **characterized in that** the connecting means (16) are designed for the pivotable bearing of the prosthesis device (2), and **in that** fixing devices (26) are provided for orienting the prosthesis device (2) relative to the prosthesis socket (1).

10. Prosthesis socket according to one of the preceding claims, **characterized in that** a cap (13) is secured releasably as a distal closure piece on the shell (10; 11, 12).

11. Prosthesis socket according to claim 1, **characterized in that** the surface configuration is formed as nap velour, oriented in distal direction.

12. Prosthesis socket according to one of the preceding claims, **characterized in that** a tensioning means (14, 15) is arranged on the shell (10; 11, 12) with two axially offset guides.

13. Prosthesis socket according to one of the preceding claims, **characterized in that** the prosthesis device (2) has a frame (20) that can be secured laterally and/or medially on the prosthesis socket (1).

14. Prosthesis socket according to Claim 13, **characterized in that** the frame (20) is designed as an outer frame.

15. System comprising a prosthesis socket (1) and a prosthesis device (2) according to one of the preceding claims.

## Revendications

1. Emboîture de prothèse destinée à recevoir un moignon d'amputation d'une extrémité, comprenant des moyens de raccordement pour un dispositif prothétique distal (2), l'emboîture de prothèse (1) comprenant au moins une coque (10 ; 11, 12) qui présente une section transversale recourbée ouverte et dont les extrémités de coque se chevauchent mutuellement au moins partiellement dans l'état appliqué, et au moins un moyen de serrage (14, 15) est agencé sur la coque (10 ; 11, 12) qui agit en direction périphérique et qui serrent les extrémités de coque l'une vers l'autre,
dans laquelle la coque (10 ; 11, 12) est réalisée en une matière plastique de forme stable ayant des zones présentant différentes élasticités,
**caractérisée en ce que**
le côté intérieur (111, 121) de la coque (11, 12) présente une configuration de surface dépendante de la direction qui, en sens opposé à une direction d'introduction du moignon d'amputation, assure une résistance supérieure à celle en direction d'introduction.

2. Emboîture de prothèse selon la revendication 1,
**caractérisée en ce que**
la coque (11, 12) est constituée par plusieurs coques partielles.

3. Emboîture de prothèse selon la revendication 1,
**caractérisée en ce que**
la coque (11, 12) est agencée de façon médiale et latérale par rapport au moignon d'amputation et comprend au moins un moyen de raccordement (16) pour le dispositif prothétique distal.

4. Emboîture de prothèse selon l'une des revendications précédentes,
**caractérisée en ce que**
la coque (10 ; 11, 12) est réalisée flexible dans la zone proximale (110, 120).

5. Emboîture de prothèse selon l'une des revendications précédentes,
**caractérisée en ce que**
la coque (10 ; 11, 12) est réalisée rigide en direction médiale et latérale.

6. Emboîture de prothèse selon l'une des revendications précédentes,
**caractérisée en ce que**
les moyens de raccordement (16) sont agencés dans la zone rigide proximale de la coque (10 ; 11, 12).

7. Emboîture de prothèse selon l'une des revendications précédentes,
**caractérisée en ce que**
les moyens de raccordement (16) sont agencés dans la zone du grand trochanter.

8. Emboîture de prothèse selon l'une des revendications précédentes,
**caractérisée en ce que**
le centre de rotation des moyens de raccordement (16) est agencé dans la zone du grand trochanter.

9. Emboîture de prothèse selon l'une des revendications précédentes,
**caractérisée en ce que**
les moyens de raccordement (16) sont réalisés pour un montage mobile en pivotement du dispositif prothétique (2),
et **en ce qu'**il est prévu des dispositifs de fixation (26) pour orienter le dispositif prothétique (2) par rapport à l'emboîture de prothèse (1).

10. Emboîture de prothèse selon l'une des revendications précédentes,
**caractérisée en ce que**
en tant qu'élément terminal distal, un capuchon (13) est fixé de façon amovible sur la coque (10 ; 11, 12).

11. Emboîture de prothèse selon la revendication 1,
**caractérisée en ce que**
la configuration de surface est réalisée en tant que velours orienté dirigé en direction distale.

12. Emboîture de prothèse selon l'une des revendications précédentes,
**caractérisée en ce que**
un moyen de serrage (14, 15) pourvu de deux guidages attaquant en décalage axial est agencé sur la coque (10 ; 11, 12).

13. Emboîture de prothèse selon l'une des revendications précédentes,
**caractérisée en ce que**
le dispositif prothétique (2) comprend un cadre qui est apte à être fixé en situation latérale et/ou médiale sur l'emboîture de prothèse (1).

14. Emboîture de prothèse selon la revendication 13,
**caractérisée en ce que**
le cadre (20) est réalisé sous forme de cadre extérieur.

15. Système constitué par une emboîture de prothèse (1) et par un dispositif prothétique (2) selon l'une des revendications précédentes.
